# EUROPEAN PATENT SPECIFICATION

(11) **EP 0 437 929 B1**
(45) Date of publication and mention of the grant of the patent: **02.03.1994**
(21) Application number: 90312713.2
(22) Date of filing: 22.11.1990
(51) Int. Cl.: A61K 31/70

(54) **Use of diadenosine-5',5'''-p1,p4-tetraphosphate for the manufacture of a medicament for the treatment of heart disease**
Verwendung von Diadenosin-5',5'''-p1,p4-tetraphosphat zur Herstellung eines Arzneimittels zur Behandlung von Herzkrankheiten
Utilisation du diadénosin-5',5'''-p1,p4-tétraphosphate pour l'obtention d'un médicament pour le traitement des maladies cardiaques

(30) Priority: 24.11.1989 JP 306092/89
(43) Date of publication of application: 24.07.1991
(73) Proprietor: FUJIREBIO INC., Tokyo 161 (JP); UNITIKA LTD., Amagasaki-shi Hyogo 660 (JP)
(72) Inventor: Inaba, Niro, Hino-shi, Toyko 191 (JP); Okamura, Kunihiro, Musashino-shi, Tokyo 180 (JP); Yamaura, Tetsuaki, Niiza-shi, Saitama 352 (JP)
(74) Representative: Woods, Geoffrey Corlett

(56) References cited:
- WO-A-89/04321
- US-A- 3 321 463
- AM. J. PHYSIOC., vol. 254, no. 5, part 2, May 1988, American Physiological Society; R. BUSSE et al., pp. H828-H832#
- EUROPEAN HEART JOURNAL, vol. 10, supplement F, 1989, European Society of Cardiology; S. NEES, pp. 28-35#
- J.E.F. REYNOLDS, "Martindale the Extra Pharmacopoeia", February 1989, The Pharmaceutical Press, London (GB)#
- BIOCHEM. JOURNAL, vol. 188, 1980; pp. 789-798#

## Description

The present invention relates to the use of a compound in the manufacture of a medicament for the treatment of arrhythmia.

There is a gradual increase in the morbidity rate due to cardiovascular diseases, such as heart disease, arteriosclerosis and hypertension, in the world as the number of old people increases and western-style diets become popular. Mortality from heart disorders ranks next to that from cancer, and is thus a real problem. Cardiac disorders commonly include arrhythmia, heart failure, angina pectoris, coronary atherosclerosis and myocardial infarction. Drugs for treating these disorders may be classified as, for example, antiarrhythmic agents, cardiotonics and coronary vasodilators.

Arrhythmia arises from abnormalities in the rhythm of the heart beat which should normally proceed at a regular pace, in particular an abnormality in the generation of spontaneous impulse in the sinoatrial node and an abnormality in the conduction system. It is classified into tachyarrhythmia and bradyarrhythmia according to the frequency of the heart beat.

Drugs for the treatment of arrhythmia include those in the following groups:
sodium ion channel inhibitors which exert an antiarrhythmic effect by inhibiting the conduction system in the His bundle, Purkinje fibers, atrium or ventricular muscle, through an electrophysiological mechanism;
β-blockers which indirectly inhibit the calcium ion channels in myocardial cell membranes, which are therefore effective in treating arrhythmia which arises from sympathicotonia or supraventricular arrhythmia in which the sinoatrial node and/or the atrioventricular node take part;
drugs which exert an antiarrhythmic effect by prolonging a duration of action potential in all myocardial cells with associated prolongation of refractory period and without the inhibition of sodium ion channels;
calcium antagonists which exert an antiarrhythmic effect by inhibiting calcium ion channels in the myocardial cell membranes through the suppression of a slow response based on a slow inward calcium current; and
other drugs such as digitalis and adenosine [Vaughan Williams, E.M., J. Clin. Pharmacol., 24, 129 (1984)].

Adenosine 5'-triphosphate (ATP), like adenosine, is known to possess antiarrhythmic activity [Somlo, E., Lancet, 268, 1125 (1955); and Komor, K. and Garas, Z., Lancet, 269, 93 (1955)] and has now attracted attention owing to its potential as a new drug for treating arrhythmia. Such an application of ATP is known to impart the following advantages: successful arrest of the paroxysmal supraventricular tachycardia; shorter time required for onset of the clinical effect of ATP; successively increasable dosage; and efficacy against paroxysmal supraventricular tachycardia which is insusceptible to other antiarrhythmic drugs such as verapamil, digoxin and ajimaline [Otsuka, F., Kagoshima, T. and Ishikawa, H., Yakkyoku, 37(4), 25-28 (1986)].

We have found that ATP itself has platelet-aggregating activity. ATP may eventually bring the risk of increasing the blood viscosity, thereby causing a disturbance in the myocardial microcirculation. Accordingly, the use of ATP as a drug for treatment of arrhythmia would be unadvisable and should be restricted in view of the above problem.

The present inventors have surprisingly found that diadenosine 5',5'''-P¹,P⁴-tetraphosphate or a pharmaceutically acceptable salt thereof can be used for the manufacture of a medicament to treat arrhythmia.

The present invention provides the use of diadenosine 5',5'''-P¹,P⁴-tetraphosphate (hereinafter referred to as Ap₄A) or a pharmaceutically acceptable salt thereof in the manufacture of a medicament for the treatment of arrhythmia.

The medicament may be in the form of a pharmaceutical composition which comprises diadenosine 5',5'''-P¹,P⁴-tetraphosphate or a pharmaceutically acceptable salt thereof and a pharmaceutically acceptable carrier or diluent.

It has been found that Ap₄A has coronary vasodilative activity and anti-platelet activity as well as ATP-like antiarrhythmic activity.

Figure 1 shows the negative inotropic effect of Ap₄A on an isolated guinea pig right atrium.

Figure 2 shows the antiarrhythmic effect of Ap₄A on a model of ouabain-induced cardiac arrhythmia.

Figure 3 shows the antiarrhythmic effect of Ap₄A on a model of adrenaline-induced cardiac arrhythmia.

Figure 4 shows the antiarrhythmic and coronary vasodilative activities of Ap₄A, wherein the abbreviations used are: P.P., perfusion pressure; H.R., heart rate; dP/dT, differential value of left ventricular pressure; and L.V.P., left ventricular pressure.

Figure 5 shows a comparison of the effect of Ap₄A with that of ATP on ADP-induced platelet aggregation. Fig. 5A depicts the effect of ATP and Fig. 5B the effect of Ap₄A.

Ap₄A is a nucleotide discovered in human platelets in recent years. It has the following chemical structure: 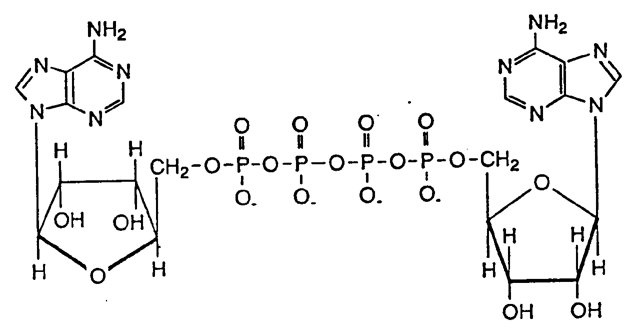
The active ingredient Ap₄A in the present invention is preferably in a form of a pharmaceutically acceptable salt thereof such as the sodium salt, or it may be in the form of the free acid. Ap₄A can be produced by conventional organic synthesis or by enzymatic synthesis with, for example, leucyl tRNA synthetase derived from thermophilic Bacillus stearothermophilus JP-A-62-278992; US-A-4886749; and EP-A-247819), using ATP as the starting material in either method. The half lethal dose (LD₅₀) of Ap₄A is 102 mg/kg (rat; i.v.) as determined by the Lichfield-Wilcoxon method. As to the biological activities of Ap₄A, there are the following reports: inhibitory effect on the adenosine diphosphate (ADP)-stimulated human platelet aggregation [Luthje, J. and Ogiluie, A., Biochem. Biophys. Res. Commun., 118, 704-709 (1984)], enhancement of DNA synthesis in baby hamster kidney cells [Grummt, F., Proc. Natl. Acad. Sci. U.S.A. 75 371-375 (1978)], promotion of the growth of hepatocytes [Rapaport, L. and Zamecnick, P.C., Proc. Natl. Acad. Sci. U.S.A., 73 3984-3988 (1976)], and vasodilative action on the rabbit mesenteric artery [Busse, R., Ogiluie, A. and Pohl, U., Am. J. Physiol., 254, 828-832 (1988)].

However, there have been no reports on the antiarrhythmic activity of Ap₄A. The present inventors have now found that Ap₄A possesses antiarrhythmic activity.

Ap₄A has an antiarrhythmic activity virtually equivalent to that of ATP, and also coronary vasodilatative activity and anti-platelet activity, Ap₄A itself has no platelet aggregation activity even at a concentration of 3x10⁻⁴M, as illustrated below. Therefore, Ap₄A is effective in the treatment of arrhythmia, and of a complication thereof. Because Ap₄A also has a prophylactic effect against thrombosis via its anti-platelet activity, in addition to the above specific activities, it is highly useful as a drug for treatment of arrhythmia.

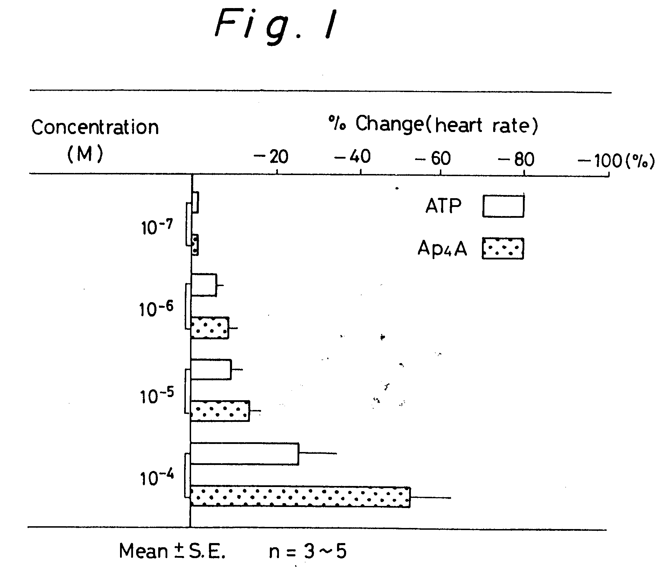

Fig. 1 shows the negative inotropic effect of Ap₄A as observed in the right atrium which was taken out from a guinea pig and exposed to Ap₄A of different concentrations, together with the results observed with ATP for comparison. The measurements of response to a single dose of the drug indicated that Ap₄A reduced the heart rate with a dose-dependency as the case of ATP and consequently showed a bradycardic effect. Such an effect of Ap₄A was maintained until Ap₄A was removed from the Ap₄A-acting system, though varying with a concentration of the drug (data not shown). It was also noted that Ap₄A was more potent than ATP, when compared in terms of the percentage change of heart rate.

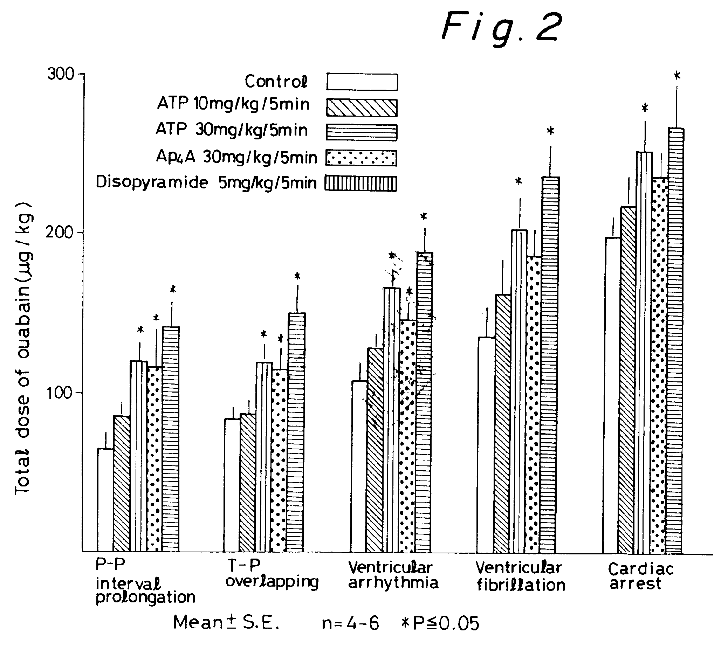

Fig. 2 illustrates the antiarrhythmic effect of Ap₄A on an ouabain-induced arrhthymia model in comparison with the effects of ATP and disopyramide. It was found that Ap₄A has an antiarrhythmic activity, as do ATP and disopyramide, as seen from the results that Ap₄A inhibited the onset of such parameters as P-P interval prolongation, T-P overlapping, ventricular arrhythmia, ventricular fibrillation and cardiac arrest. The antiarrhythmic activity of Ap₄A was practically the same as that of ATP, or slightly lower, when compared at the same concentration.

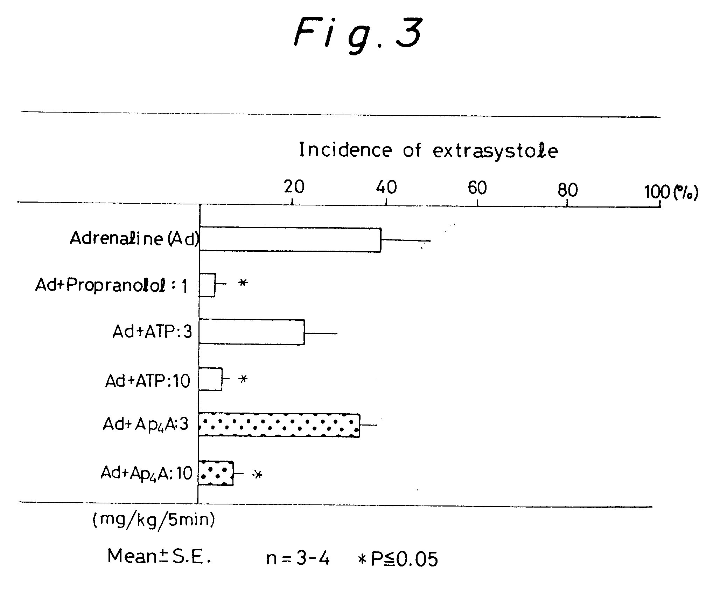

The antiarrhythmic effect of Ap₄A on an adrenaline-induced arrhythmia model is compared with that of ATP or propranolol in Fig. 3. When the grading of arrhythmia was assessed in terms of the incidence of extrasystole, Ap₄A reduced the incidence of extrasystole with a dose-dependency as did ATP and propranolol. This is evidence which corroborates the antiarrhythmic effect of Ap₄A. The effect of Ap₄A was somewhat inferior to that of ATP.

Thus, the antiarrhythmic activity of Ap₄A was verified in the two types of cardiac arrhythmia models induced with ouabain and adrenalin. It was found that the antiarrhythmic efficacy of Ap₄A is analogous to ATP. From the two experiments on these models, it is inferred that the antiarrhythmic effect of Ap₄A is probably exerted via its ability to produce a bradycardic response by inhibiting the sinoatrial node, which mechanism is also the case for ATP.

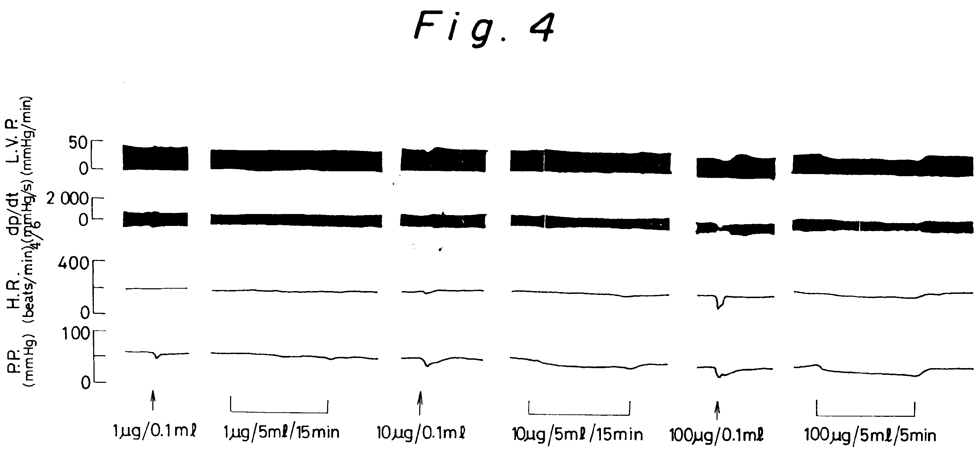

Fig. 4 shows a change of perfusion pressure observed when the guinea pig heart was removed and placed under Langendorff perfusion and Ap₄A was then added to the perfusion system in a single or continuous dose. In either method of administration of Ap₄A, the perfusion pressure decreased depending on the dose. This finding indicates that Ap₄A possesses coronary vasodilative activity as well as antiarrhythmic activity. The coronary vasodilative effect was demonstratable at the dose which did not affect the heart rate or left ventricular pressure, e.g., 1 µg. Thus, Ap₄A is expected to be a highly potent coronary vasodilative agent.

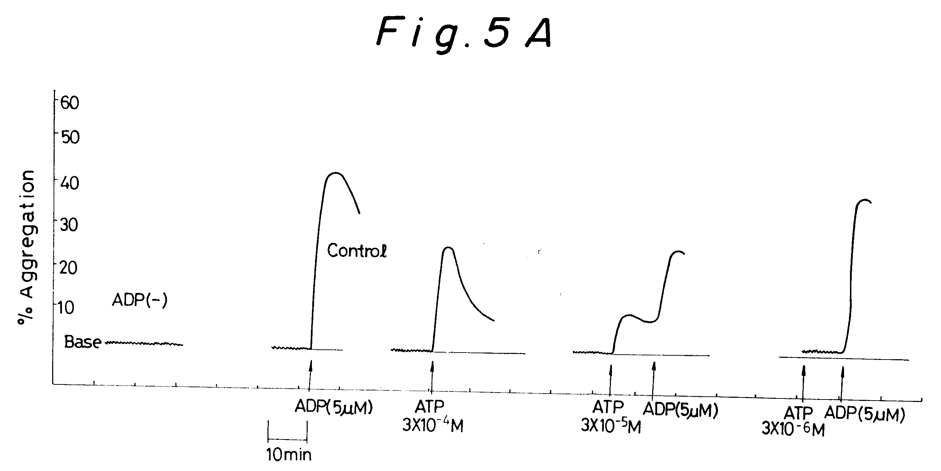

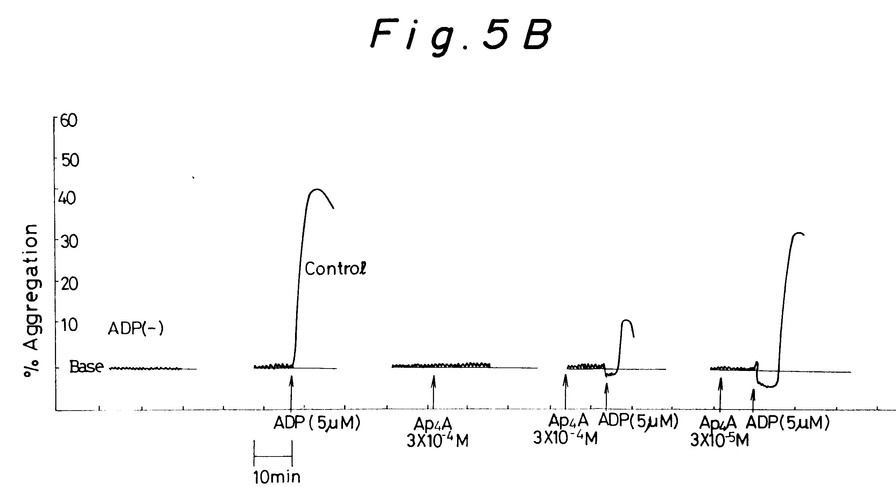

In Fig. 5, the inhibitory effect of Ap₄A on ADP-induced platelet aggregation is compared with that of ATP. ATP which was administered at a concentration of 3x10⁻⁵ M or more in the absence of ADP caused a dose-dependent aggregation of platelets, while Ap₄A did not cause platelet aggregation at all under the same conditions. When Ap₄A was added to the platelet-rich plasma derived from a rabbit at a concentration of, for example, from 3x10⁻⁵ M to 3x10⁻⁴ M, prior to addition of ADP (5µM), Ap₄A markedly inhibited ADP-induced platelet aggregation with a dose-dependency, as described by J. Luthje and A. Ogiluie (see the above cited reference). For example, Ap₄A at 3x10⁻⁴ M inhibited the platelet aggregation by 76%.

As stated hereinabove, Ap₄A has antiarrhythmic activity analogous to ATP and at the same time has coronary vosodilation and anti-platelet aggregation activities. Therefore, use of Ap₄A in the manufacture of a medicament for the treatment of arrhythmia avoids the problem of disturbance of myocardial microcirculation caused by the platelet aggregation inherent in ATP itself.

The pharmaceutical composition comprising Ap₄A may be administered orally or by injection, e.g. intravenously or intramuscularly. The pharmaceutical composition may be administered at an effective dosage level of Ap₄A of, for example, 10-2000 mg/day/adult orally or 1-500 mg/day/adult intravenously. A patient with arrhythmia may have administered orally divided doses intravenously or a slow infusion over a certain length of time. The dosage and method of administration may be individualized, for example according to the patient's age, symptom and body weight.

In the pharmaceutical composition a therapeutically effective amount of Ap₄A is combined with an inert carrier or diluent. Conventional carriers or diluents may be used and the pharmaceutical composition may further contain one or more pharmaceutically acceptable additives such as preservatives, stabilizers and perfumes. The pharmaceutical composition may be prepared in the form of, for example, granules, a tablet, capsule, injection or parenteral infusion fluid. Its form is generally selected according to patient's age, symptoms and other factors.

The present invention is now further illustrated in the following Examples.

Example 1

### (A) Effect of Ap₄A on guinea pig right atrium

A guinea pig (b.w. 450g) was stunned and its heart was immediately removed and the right atrium was excised therefrom. The right atrium was then suspended in a Magnus tube (30 ml) filled with Krebs-Henseleit (KH) solution which was maintained at 31°C, constantly aerating with a mixture of 95% O₂ - 5% CO₂. The KH solution was changed with fresh ones at 15-minutes intervals. The heart rate of the right atrium was stabilized in 60-90 minutes, and the Ap₄A or ATP, which had been prepared at different concentration, was added to the KH solution in a single dose to determine the change of heart rate. Drug concentrations of 10⁻⁴, 10⁻⁵, 10⁻⁶ and 10⁻⁷M were employed. The heart rate was determined with a cardiotachometer (Nihon Kohden Co.; Model AT601G; Japan) and an isometric converter (Nihon Kohden Co.; Model TB-651T), with a 1-g weight load. The results are shown in Fig. 1.

As seen in Fig. 1, both Ap₄A and ATP decreased the atrial heart rate with a dose-dependency. This means that these drugs have a bradycardic effect. When their changes of heart rate are compared, the potency of Ap₄A tends to be greater than that of ATP; the relative potency of Ap₄A was 1.5-fold at 10⁻⁶M, 1.5-fold at 10⁻⁵M and 2.1-fold at 10⁻⁴M.

### (B) Effect of Ap₄A on ouabain-induced arrhythmia

With a guinea pig (b.w. 450g) placed under urethane anesthesia (1.1 g/kg), its femoral vein, jugular vein and carotid artery were cannulated. Ap₄A, ATP or disopyramide was injected into the femoral vein and ouabain into the jugular vein through each cannula, while the carotid artery was utilized for monitoring blood pressure. Changes associated with the administration of the drug were assessed by monitoring electrocardiogram (ECG), blood pressure, mean blood pressure and heart rate (Nihon Kohden Co.; Models AB620G, AP601G and AT601G). The ECG was recorded in accordance with lead II.

The ouabain-induced cardiac arrhythmia model was prepared by continuous infusion of ouabain at a rate of 7 µg/kg/min via the jugular vein. Each drug was administered by intravenous infusion over a period of 5 minutes in the following doses : ATP, 10 or 30 mg/kg; Ap₄A, 30 mg/kg; and disopyramide, 5 mg/kg. The drug was administered simultaneously with the infusion of ouabain. The antiarrhythmic effect of the drug tested was assessed in terms of the total dose of ouabain given until onset of each of the following parameters: P-P interval prolongation, T-P overlapping, ventricular arrhythmia, ventricular fibrillation and cardiac arrest. The results are shown in Fig. 2.

The data indicated a marked antiarrhythmic effect of Ap₄A, which inhibited all these parameters, as did ATP and disopyramide. The antiarrhythmic effect of Ap₄A at a dose of 30 mg/kg was virtually comparable with or slightly inferior to that of ATP at the same dose.

### (C) Effect of Ap₄A on adrenaline-induced arrhythmia

With a guinea pig (b.w. 450g) placed under urethane anesthesia (1.1 g/kg), its femoral vein, jugular vein and carotid artery were cannulated. Ap₄A, ATP or propranolol was injected into the femoral vein and adrenaline into the jugular vein through each cannula, while the carotid artery was utilized for monitoring blood pressure. Changes associated with the administration of the drug were monitored in the same manner as above (B), and the ECG was recorded in accordance with lead II.

Adrenaline-induced cardiac arrhythmia model was prepared by injecting adrenaline (10 µg/kg) into the jugular vein over 5 seconds. Each drug was administered by intravenous infusion over a period of 5 minutes at the following doses: ATP, 3 or 10 mg/kg; Ap₄A, 3 or 10 mg/kg; propranolol, 1 mg/kg. Adrenaline was administered in a single dose 4 minutes after administration of the drug tested. The grading of arrhythmia was assessed in terms of the incidence of extrasystole. The results are shown in Fig. 3.

Ap₄A exhibited an antiarrhythmic effect with a dose-dependent reduction of the incidence of extrasystole, as did ATP and propranolol. The effect of Ap₄A was somewhat weaker than that of ATP. For example, the incidence of extrasystole at a dose of 10 mg/kg/5min was 5.3% with ATP and 8.0% with Ap₄A, as compared to a control value of 39%.

The following examples 2 and 3 are for purpose of illustration and do not fall within the scope of the claims.

### Example 2

### Coronary vasodilative effect

An experiment was performed by a modification of the method of Aronson and Serlick [Toxicol. Appl. Pharmac., 38, 479-488 (1976) and 39, 157-176 (1977); Biochem. Pharmac. 26, 2297-2305 (1977)]. A guinea pig heart was removed and perfusing at the origin of the aorta was started by the Langendorff perfusion method. Perfusion was carried out at 37°C and 14 ml/min flow rate with a peristalic pump (TAIYO TYPE 1500N; Japan) in Krebs-Henseleit solution (KH: NaCl, 118mM; phosphate, 1.2mM; NaHCO₃, 25mM; glucose, 10mM; KCl, 4.7mM; CaCl₂, 2.5mM; and MgSO₄, 1.2mM; pH 7.4; bubbling with 95% O₂ - 5% CO₂). After the initiation of the perfusion, an incision was made in the left auricle and a cannula was inserted into the left ventricle via the left atrium to measure the left ventricular pressure with a pressure transducer (Nihon Kohden Co.; Model TP-200T). The differential value of the left ventricular pressure (dp/dt) was measured with a cardiotachometer (Nihon Kohden Co.; Model AT-601G). The perfusion pressure was determined with a pressure transducer connected to the aortic cannula. Ap₄A was dissolved in KH at various concentrations and administered to the perfusion system in a single dose of 1-100 µg/0.1ml/5s or by a continuous infusion of 1-100 µg/5ml/15min. The results are shown in Fig. 4.

The data indicate a dose-dependent fall of perfusion pressure and a marked decrease of heart rate after the single dose of Ap₄A. In addition, the left ventricular pressure fell transiently after administration and subsequently it was elevated. The effect of Ap₄A on the perfusion pressure was observed from the lowest dose of Ap₄A. In the continuous infusion of Ap₄A, the perfusion pressure and the left ventricular pressure decreased depending on the dose of Ap₄A. The decrease of perfusion pressure clearly indicates that Ap₄A has coronary vasodilative activity.

### Example 3

### Effect of Ap₄A on ADP-induced platelet aggregation

Blood was collected from the carotid artery of an unanesthetized rabbit into a syringe containing one volume of 3.8% sodium citrate per nine volumes of blood. The citrated blood was centrifuged at 1100 rpm for 15 minutes, and the supernatant was obtained as platelet-rich plasma (PRP). To 200 µl of PRP in an aggregometer, 10 µl of ATP or Ap₄A solution was added and the mixture was incubated at 37°C for 10 minutes, followed by addition of 10 µl of 0.1mM ADP solution (final concentration: 5µM) to induce and measure the aggregation of platelets. The results obtained for ATP are shown in Fig. 5A and those for Ap₄A in Fig. 5B.

As seen in Fig. 5B, ADP-induced platelet aggregation was inhibited by Ap₄A with a dose-dependency, namely by 76% at 3x10⁻⁴M and 21% at 3x10⁻⁵M, as compared to the control. In addition, Ap₄A itself had no effect on the platelet aggregation even at a concentration of 3x10⁻⁴M. ATP caused a dose-dependent aggregation of platelets at concentrations of 3x10⁻⁵M or more (Fig. 5A).

## Claims

1. Use of diadenosine 5',5'''-P¹,P⁴-tetraphosphate or a pharmaceutically acceptable salt thereof in the manufacture of a medicament for the treatment of arrhythmia.

2. Use according to claim 1 wherein the diadenosine 5',5'''-P¹,P⁴-tetraphosphate is in the form of a sodium salt thereof.

3. Use according to claim 1 or 2 wherein the medicament is in the form of a composition suitable for oral administration or injection.

## Patentansprüche

1. Verwendung von Diadenosin 5',5'''-P¹,P⁴-tetraphosphat oder einem pharmazeutisch verträglichen Salz hiervon bei der Herstellung eines Arzneimittels zur Behandlung von Arrhythmie.

2. Verwendung nach Anspruch 1, wobei das Diadenosin 5',5'''-P¹,P⁴-tetraphosphat in Form eines Natriumsalzes hiervon vorliegt.

3. Verwendung nach Anspruch 1 oder 2, wobei das Arzneimittel in Form einer Zusammensetzung vorliegt, die für die orale Verabreichung oder eine Injektion geeignet ist.

## Revendications

1. Utilisation du diadénosin-5',5'''-P¹, P⁴-tétraphosphate ou d'un sel pharmaceutiquement acceptable de celui-ci pour l'obtention d'un médicament destiné au traitement de l'arythmie.

2. Utilisation selon la revendication 1 dans laquelle le diadénosin-5',5'''-P¹, P⁴-tétraphosphate est sous forme d'un sel de sodium.

3. Utilisation selon la revendication 1 ou 2 dans laquelle le médicament est sous forme d'une composition convenant à l'administration par voie orale ou injectable.
